# EUROPEAN PATENT APPLICATION

(11) **EP 4 481 393 A1**
(43) Date of publication of application: **25.12.2024**
(21) Application number: 23774763.9
(22) Date of filing: 16.03.2023
(51) Int. Cl.: G01N 35/00, G01N 35/02, G01N 35/10

(54) **INSPECTION DEVICE**

(30) Priority: 22.03.2022 JP 2022046016
(71) Applicant: FUJIFILM Corporation, Tokyo 106-8620 (JP)
(72) Inventor: MIURA, Yoshinobu, Ashigarakami-gun, Kanagawa 258-8538 (JP); HIBE, Daisuke, Ashigarakami-gun, Kanagawa 258-8538 (JP)
(74) Representative: Dehns Germany Partnerschaft mbB
(86) International application number: PCT/JP2023/010397
(87) International publication number: WO 2023/182167

(57) **Abstract**

An examination apparatus examines a specimen using a liquid container which accommodates a liquid and of which an opening portion is sealed with a sealing film. The examination apparatus includes: a perforating mechanism that perforates the sealing film with a perforating tool; and a camera that captures an image of a scattered state of the liquid on a front surface of the sealing film in a case where the sealing film is perforated with the perforating tool, and outputs a captured image.

## Description

### BACKGROUND OF THE INVENTION

### 1. Field of the Invention

The technology of the present disclosure relates to an examination apparatus.

### 2. Description of the Related Art

An examination apparatus that performs an examination using a biological sample such as blood as a specimen is known (refer to WO2016/139997A). In the examination apparatus disclosed in WO2016/139997A, a liquid container, which includes a well-shaped accommodating portion that accommodates a liquid such as a reagent and a lid that closes an opening portion formed in an upper portion of the accommodating portion, is used in the examination. The examination apparatus is provided with a perforating tool that pierces and perforates the lid of the liquid container.

### SUMMARY OF THE INVENTION

As in the examination apparatus described in WO2016/139997A, in a case where the liquid container is perforated with the perforating tool, the liquid may be scattered. In a case where the liquid scatters, adverse effects, such as contamination and pollution of the liquid, occur. Although a countermeasure for such a problem has been studied, the liquid scattering occurs inside the examination apparatus. Therefore, there is a problem in that it is difficult to ascertain the problem caused by the liquid scattering in the first place.

The technique according to the present disclosure provides an examination apparatus capable of ascertaining the adverse effects of liquid scattering.

According to an aspect of the present disclosure, there is provided an examination apparatus that examines a specimen using a liquid container which accommodates a liquid and of which an opening portion is sealed with a sealing film, the examination apparatus comprising: a perforating mechanism that perforates the sealing film with a perforating tool; and a camera that captures an image of a scattered state of the liquid on a front surface of the sealing film in a case where the sealing film is perforated with the perforating tool, and outputs a captured image.

The examination apparatus may further comprise a processor that is configured to derive the scattered state of the liquid by analyzing the image.

The processor may be configured to determine a risk of liquid contamination on the basis of the scattered state.

The processor may be configured to output a determination result of the risk.

The processor may be configured to issue a warning that an examination result of the specimen is an error in a case where a degree of the risk is greater than a preset degree.

The processor may be configured to perform a control of changing an operation of the perforating mechanism in a case where a degree of the risk is greater than a preset degree.

The processor may be configured to perform, as the control of changing the operation of the perforating mechanism, a control of changing a suction amount or a discharge amount of the liquid in a case where the perforating tool is a nozzle that performs suction and discharge of the liquid.

The liquid container may have a plurality of accommodating portions that accommodate a plurality of types of liquids as the liquid.

The liquid may include at least one of the specimen or a reagent which is used for examination of the specimen.

The reagent may include a plurality of types of reagents.

An examination target substance included in the specimen may be detected by using an antigen-antibody reaction.

According to the technology of the present disclosure, it is possible to ascertain the adverse effects which are caused by the scattering of the liquid.

### BRIEF DESCRIPTION OF THE DRAWINGS

Fig. 1 is a schematic diagram showing an overall configuration of an examination apparatus.
Fig. 2 is a perspective view of a cartridge.
Fig. 3 is a diagram showing an image obtained by capturing a scattered state of a liquid.
Fig. 4 is an explanatory diagram of a determination condition table.
Fig. 5 is a diagram showing an example of a display screen of a determination result.
Fig. 6 is a flowchart showing a process procedure of risk determination.
Fig. 7 is a diagram showing another example of the display screen of the determination result.
Fig. 8 is a flowchart showing a control of changing an operation of a dispensing mechanism.

### DESCRIPTION OF THE PREFERRED EMBODIMENTS

Hereinafter, an examination apparatus according to an embodiment of the present disclosure will be described with reference to the drawings.

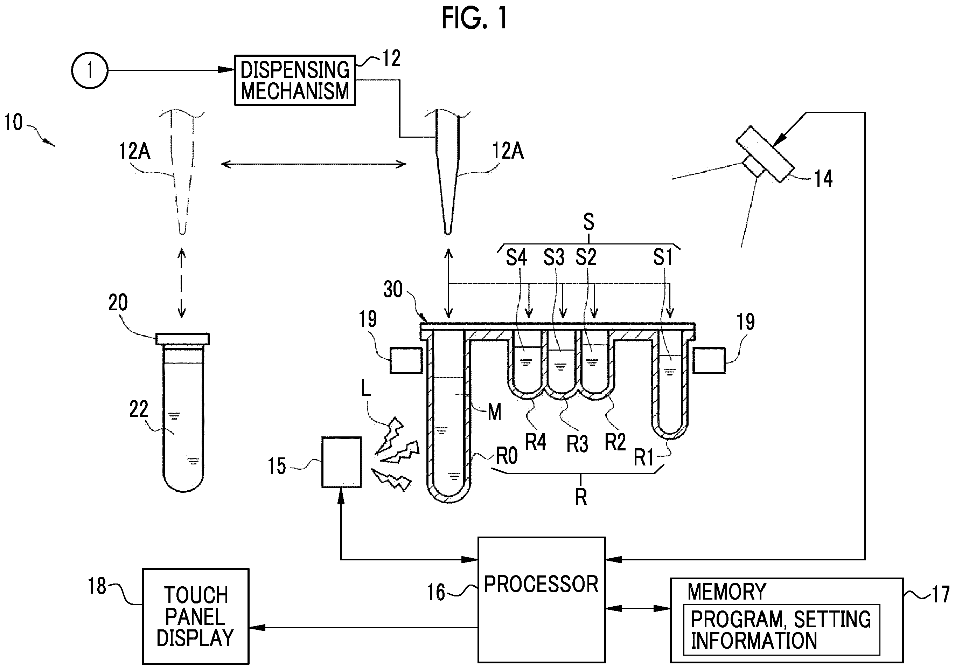

Fig. 1 is a schematic view showing an overall configuration of an examination apparatus 10 according to the embodiment of the present disclosure. For example, the examination apparatus 10 is an immunological analysis apparatus that detects an examination target substance in a specimen 22 collected from a biological body by using an antigen-antibody reaction. The examination apparatus 10 optically detects the examination target substance in the specimen 22 by using a cartridge 30 and outputs an examination result.

The cartridge 30 has a plurality of cells R including a reaction cell R0 in which the specimen 22 and a reagent S are reacted with each other, and cells R1 to R4 for respectively accommodating a plurality of types of reagents S1 to S4 necessary for the examination. The cartridge 30 is an example of a liquid container according to the technology of the present disclosure, and the plurality of cells R are an example of an accommodating portion that accommodates the liquid. The cartridge 30 is attachably and detachably loaded into the examination apparatus 10. The cartridge 30 is a single-use type that is used once for one specimen 22. For example, the cartridge 30 includes all the reagents S necessary for the examination of the specimen 22.

The specimen 22 is, for example, a biological fluid such as blood collected from a biological body. In a case where the specimen 22 is blood, the specimen 22 may be any of whole blood, blood plasma, serum, and the like. Further, a centrifuge may be provided in the examination apparatus 10 to extract blood plasma or serum from whole blood. Furthermore, the examination target substance that can be included in the specimen 22 is an antigen, an antibody, a protein, a low-molecular-weight compound, and the like. It should be noted that the specimen 22 is not limited to blood, and may be a substance collected from a biological body, such as urine or body fluid.

The examination apparatus 10 of the present example performs an examination based on a chemiluminescent enzyme immunoassay method. The reagent S accommodated in the cartridge 30 is four types of reagents, that is, the reagents S1 to S4 respectively accommodated in the cell R1 to the cell R4. For example, the reagent S1 is a buffer solution, the reagent S2 is a labeling reagent, the reagent 3 is a first luminescent reagent, and the reagent S4 is a second luminescent reagent. In addition, although not shown in the drawing, a binding substance that specifically binds to the examination target substance through the antigen-antibody reaction with the examination target substance is fixed in advance in the reaction cell R0.

The chemiluminescent enzyme immunoassay method of the present example will be simply described later. The specimen 22 is discharged to the reaction cell R0 to mix the specimen 22 and the binding substance fixed in advance. In a case where the specimen 22 includes the examination target substance, the binding substance that has been fixed in advance in the reaction cell R0 binds to the examination target substance in the specimen 22. The reagent S2, which is a labeling reagent, also has a binding substance that specifically binds to the examination target substance, and in a case where the specimen 22 includes the examination target substance, the examination target substance is labeled the binding to the reagent S2. The label included in the reagent S2 is an enzyme, and the reagent S2 is chemiluminescent in a case of presence of the reagent S3 which is a first luminescent reagent and the reagent S4 which is a second luminescent reagent. In such a manner, in the chemiluminescent enzyme immunoassay method, the examination target substance is detected through chemiluminescence by a chemical reaction between the reagent S2 bound to the examination target substance and the reagents S3 and S4.

Hereinafter, in a case where it is not necessary to distinguish each of the plurality of the reaction cell R0 and the cells R1 to R4 and each of the plurality of reagents S1 to S4, the cell and the reagent will be simply referred to as a cell R and a reagent S. Further, in Fig. 1, a reference numeral M represents a mixed liquid of the specimen 22 and the reagent S.

For example, the examination apparatus 10 includes a dispensing mechanism 12, a camera 14, a detecting unit 15, a processor 16, a memory 17, a touch panel display 18, and a loading section 19.

The loading section 19 allows the cartridge 30 to be attachably and detachably loaded thereinto and holds the cartridge 30 in the examination apparatus 10.

The detecting unit 15 executes a detecting process of detecting the examination target substance in the specimen 22. In the present example, the detecting unit 15 is composed of a light receiving section such as a photomultiplier tube or a photodiode. The detecting unit 15 detects the examination target substance bound to the reagent S2, which is a labeling reagent, optically by receiving light L emitted through chemiluminescence. For example, the detecting unit 15 is disposed to face the reaction cell R0, and receives the light L emitted by the mixed liquid M which is obtained by mixing the specimen 22 and the reagent S. The detecting unit 15 outputs a received light signal corresponding to an amount of received light to the processor 16.

A processor 16 integrally controls each unit of the examination apparatus 10. An example of the processor 16 is a central processing unit (CPU) that performs various types of control by executing a program. The CPU functions as a control unit that controls each unit by executing a program. Further, the processor 16 detects whether or not the specimen 22 includes the examination target substance and the concentration thereof on the basis of the received light signal which is output by the detecting unit 15. Further, as will be described later, the processor 16 also executes a risk determination process of determining a risk of contamination due to unintended mixing of a plurality of types of liquids to be accommodated in the cartridge 30, using the camera 14.

A memory 17 is an example of a memory connected to or built into the CPU as the processor 16. For example, the memory 17 stores various programs such as a program for controlling the examination apparatus 10 and a program for executing the detecting process. Further, the memory 17 stores setting information used by the processor 16 to perform various controls, in addition to a control program.

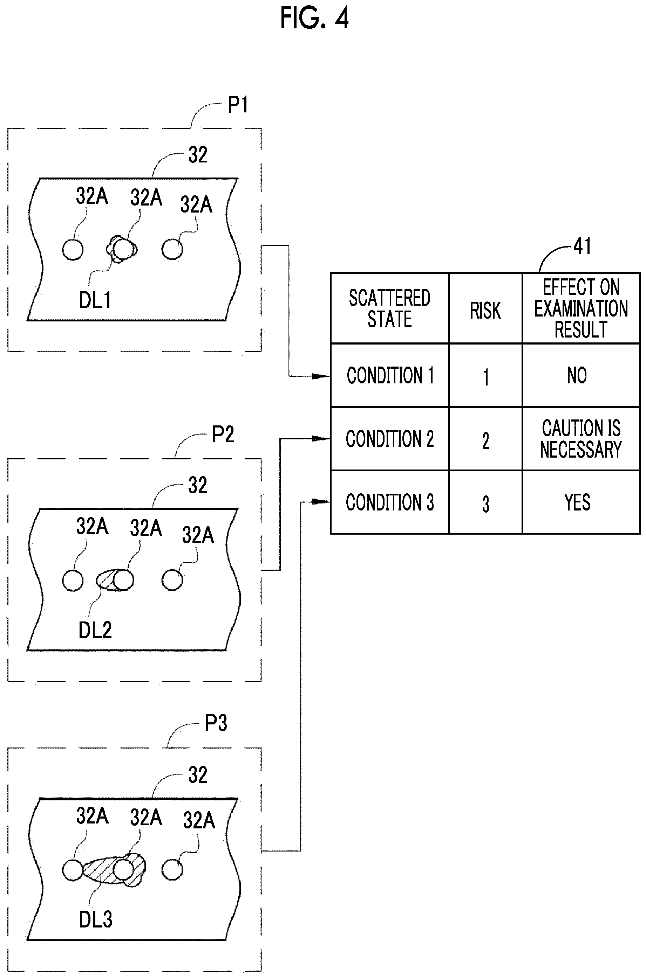

The setting information includes, for example, information indicating a correspondence relationship between a light amount of the light L and an amount of the examination target substance and a determination condition table 41 (refer to Fig. 4) used for determining the risk of contamination.

The touch panel display 18 receives an operation instruction, such as an instruction to start an examination, from a user. Further, the touch panel display 18 displays various types of information such as an examination result and a determination result of the risk of contamination. As described above, the touch panel display 18 functions as an operation instruction receiving unit that receives the operation instruction and the display unit that displays various types of information.

The dispensing mechanism 12 is a mechanism that dispenses a liquid, and includes a nozzle 12A that suctions and discharges the liquid, a moving mechanism (not shown in the drawing), and a suction-discharge mechanism (not shown in the drawing). The moving mechanism is a mechanism that three-dimensionally moves the nozzle 12A in a vertical direction and a horizontal direction. The suction-discharge mechanism is a mechanism that causes the nozzle 12A to perform a suction operation of suctioning the liquid and a discharge operation of discharging the liquid from the nozzle 12A. The moving mechanism is composed of an actuator that generates a driving force, such as a motor, and a driving force transmission mechanism consisting of a gear, a drive belt, and the like. The suction-discharge mechanism is composed of a pump or the like that generates a driving force for suction and discharge.

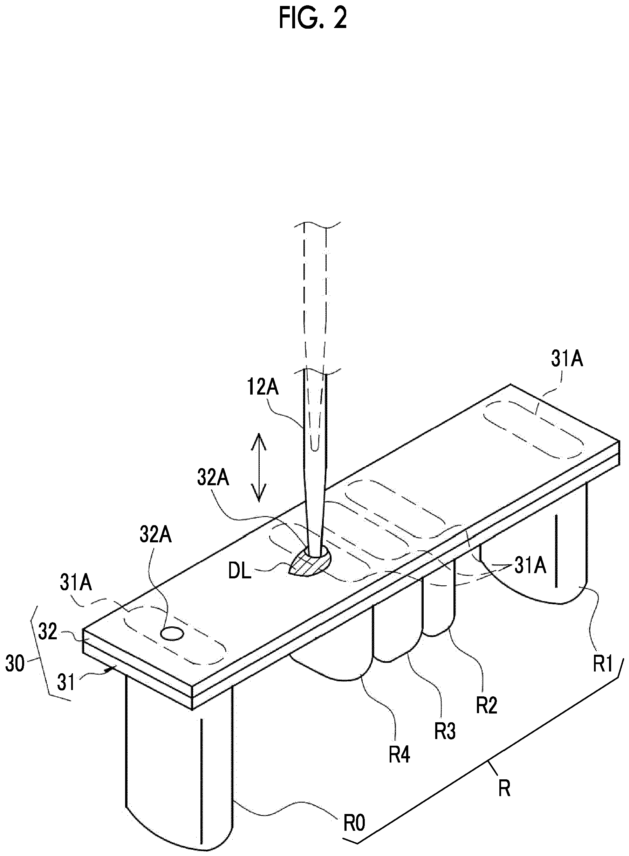

The dispensing mechanism 12 performs suctioning and dispensing of the liquid such as the specimen 22 and the reagent S by using the nozzle 12A. Specifically, the dispensing mechanism 12 first discharges the reagent S (in the present example, the buffer solution S1) included in the cell R1 to the reaction cell R0. Next, the dispensing mechanism 12 suctions the specimen 22 from the specimen collection container 20 which accommodates the specimen 22 before the start of the examination, and discharges the suctioned specimen 22 to the reaction cell R0 of the cartridge 30, thereby dispensing the specimen 22 to the reaction cell R0. Then, the dispensing mechanism 12 suctions the plurality of reagents S accommodated in the cartridge 30 and discharges the suctioned reagents S to the reaction cell R0 and the like to dispense the reagents S. In addition, the dispensing mechanism 12 executes a mixing process of mixing the specimen 22 and the reagent S in the reaction cell R0 or the like. The mixing process is performed, for example, by repeating suction and discharge of the mixed liquid through the nozzle 12A. It should be noted that in Figs. 1 and 2, for convenience, the nozzles used for the specimen 22 and the reagent S are represented by one nozzle 12A. However, in reality, the dispensing mechanism 12 includes the nozzle for the specimen used for the specimen 22 and the nozzle for the reagent used for the reagent S, and the nozzles are used selectively.

The camera 14 is disposed above the loading section 19 of the cartridge 30. The camera 14 captures an image of the scattered state of the liquid in the cartridge 30 from above the cartridge 30 and outputs a captured image P as an image which has been captured. The processor 16 determines the risk of contamination with the liquid on the basis of the captured image P. The camera 14 is an optical camera, and includes an image sensor that captures an image of a scattered state of the liquid, and an optical system that forms the scattered state of the liquid on an image formation surface of the image sensor. The image sensor is a complementary metal oxide semiconductor (CMOS) image sensor, a charge coupled device (CCD) image sensor, or the like.

As shown in Fig. 2, the cartridge 30 includes a body 31, which has the cells R, and a sealing film 32. The body 31 is made of, for example, plastic, and the cells R are integrally formed therein. Each cell R has a well shape in which an opening portion 31A is formed on the upper portion. The sealing film 32 is made of, for example, aluminum, is provided on the upper surface of the body 31, integrally covers the opening portions 31A of the cells R, and seals the opening portions 31A of the cells R. In the cartridge 30 of the present example, the three cells R including the cells R2 to R4 are disposed to be adjacent to each other in the center, and the cell R0 and the cell R1 are disposed with relatively large intervals between the three cells R2 to R4. Therefore, the intervals between the opening portions 31A of the cells R2 to R4 are relatively narrow, as compared with the intervals between the opening portions 31A of the cells R0 and R4 and the interval between the opening portions 31A of the cells R2 and R1.

In addition to suctioning and discharging of the liquid, the nozzle 12A pierces the sealing film 32 by moving downward from above the sealing film 32 toward a front surface of the sealing film 32 at a position of each opening portion 31A as shown in Fig. 2, thereby forming a hole 32A in the sealing film 32. That is, the nozzle 12A is an example of a "perforating tool" according to the technology of the present disclosure, and the dispensing mechanism 12 is an example of a "perforating mechanism".

The liquid such as the reagent S may be attached to the rear surface (surface facing the opening portion 31A) of the sealing film 32. In a case where the nozzle 12A pierces the sealing film 32, the liquid attached to the rear surface of the sealing film 32 may be scattered around the hole 32A on the front surface of the sealing film 32 with the movement of the nozzle 12A at the time of pulling out the nozzle 12A from the hole 32A. Further, since the nozzle 12A comes into contact with the liquid in the cell R, the liquid is attached to the distal end of the nozzle 12A. In a case where the nozzle 12A is pulled out from the hole 32A, the attached liquid may be scattered around the hole 32A on the front surface of the sealing film 32. In Figs. 2 and following figures, a liquid DL represented by a reference numeral DL indicates liquid droplets of the liquid scattered around the holes 32A on the front surface of the sealing film 32, and indicates liquid droplets of a liquid including at least one of the specimen 22 or the reagent S.

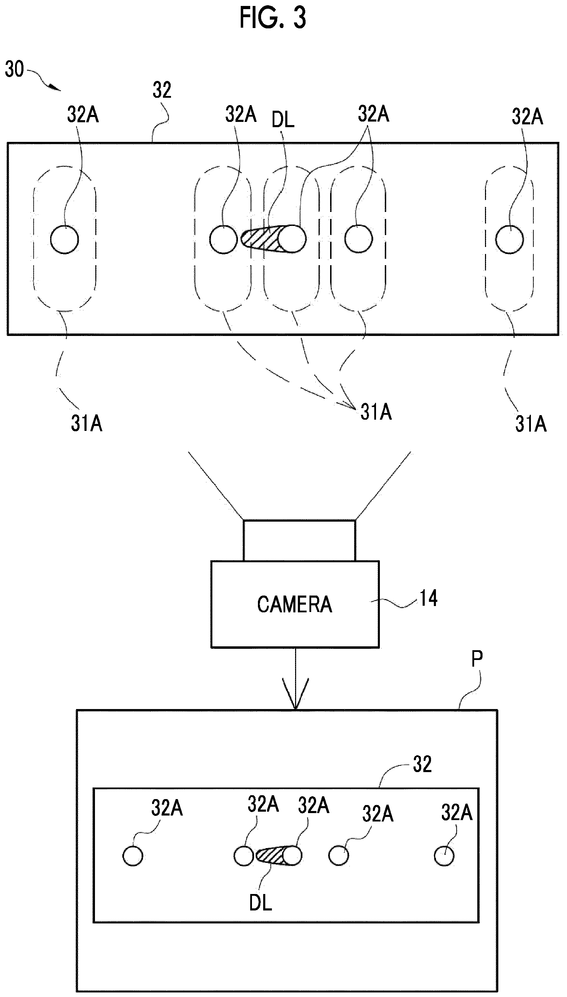

As shown in Fig. 3, the camera 14 captures an image of the scattered state of the liquid DL on the front surface of the sealing film 32 of the cartridge 30, and outputs the captured image P as an image which has been captured. The processor 16 derives the scattered state of the liquid DL by analyzing the captured image P. For example, the processor 16 extracts a contour of the scattered liquid DL in the captured image P, and derives, as the scattered state, an area of the scattered liquid DL, an interval between the adjacent hole 32A and the scattered liquid DL, and the like.

In the present example, the processor 16 determines the risk of the contamination of the liquid DL, on the basis of the derived scattered state of the liquid DL. The contamination refers to, for example, unintended mixing of the plurality of types of liquids, such as unintended mixing of the reagent S1 accommodated in the cell R1 and the reagent S2 accommodated in the cell R2. Since the suction amount and the discharge amount of the reagent S affect the examination result, the dispensing mechanism 12 manages the reagent S at an appropriate value. Therefore, for example, in a case where the reagent S2 is mixed into the cell R1 accommodating the reagent S1 different therefrom due to the scattering of the liquid DL, an amount of the reagent S1 managed at the appropriate value may unintendedly deviate from the appropriate value. In a case where the amount of the reagent S deviates from the appropriate value, for example, the amount of luminescence is increased to be equal to or higher than the concentration of the examination target substance. In such a case, there is a problem in that there is an adverse effect on the examination result, for example, in that the concentration of the examination target substance cannot be accurately detected. The effect on such an examination result is a risk of contamination.

As shown in Fig. 4, the memory 17 stores the determination condition table 41 used by the processor 16 to determine the risk of contamination, as the setting information. The determination condition table 41 is a table that defines conditions for determining which degree of the risk of contamination should be evaluated for the scattered state of the liquid DL which is derived from the captured image P. The determination condition table 41 defines a correspondence relationship between the scattered state of the liquid DL, a degree of the risk corresponding to the scattered state, and the effect on the examination result corresponding to the degree of the risk. For example, in the determination condition table 41, three-stage risks of "level 1" to "level 3" are defined as the degrees of the risk. The level 1 means that there is no effect on the examination result and the risk is almost zero. The level 2 means that the contamination is likely to slightly occur and that caution is necessary for evaluating the examination result. The level 3 means that the contamination reliably occurs and has an effect on the examination result. The level 3 is a level at which the examination result is unreliable and the examination result should be determined as an error.

In the determination condition table 41, the scattered state derived from the captured image P is defined as the conditions 1 to 3 in association with the respective degrees of the risks of the levels 1 to 3. For example, the conditions 1 to 3 of the scattered state are preset on the basis of an area of the scattered liquid DL appearing in the captured image P1, the intervals between the adjacent holes 32A, and the like.

In Fig. 4, for example, the condition 1 is a condition which is set by assuming the scattered state of the liquid DL1 shown in the captured image P1. In the captured image P1, the liquid DL1 is scattered around the hole 32A. However, an area thereof is relatively small as compared with the liquid DL2 and the liquid DL3 of the other captured images P2 and P3, and the interval between the liquid DL and the hole 32A adjacent thereto is relatively large.

For example, the condition 3 is a condition which is set by assuming a scattered state of the liquid DL3 shown in the captured image P3. An area of the liquid DL3 is relatively large as compared with the areas of the liquids DL1 and DL2 of the other captured images P1 and P2, and the interval between the liquid DL3 and the hole 32A adjacent thereto is almost zero. The scattered state shown in the captured image P3 is likely to be a state where contamination occurs.

For example, the condition 2 is a condition which is set by assuming the scattered state of the liquid DL2 shown in the captured image P2. The liquid DL2 is the scattered state that is positioned substantially in the middle of the liquid DL1 and the liquid DL3. Specifically, in a case of the liquid DL2, the interval between the scattered liquid DL and the hole 32A adjacent thereto is within a half of the interval between the holes 32A adjacent to each other. In such a case, the contamination is likely to slightly occur. However, the contamination may not have a significant effect on the examination result to evaluate the examination result as an error.

The processor 16 determines the degree of risk in the levels 1 to 3 by checking which of the conditions of the determination condition table 41 the scattered state of the liquid DL derived from the captured image P corresponds to. The processor 16 outputs the determination result in the levels 1 to 3 to the touch panel display 18 to present the determination results to the user. Further, the processor 16 outputs, as the information indicating the degree of each risk, the effect of the examination result defined in the determination condition table 41 in addition to the degree of risk in the levels 1 to 3.

For example, in a case where the degree of the risk is the level 1, the processor 16 outputs a message such as "There is no risk of contamination, and there is no effect on the current examination result". In a case where the degree of the risk is the level 2, the processor 16 outputs a message such as "There is a slight risk of contamination, and caution is necessary for the current examination result". In a case where the degree of the risk is the level 3, a message such as "There is a high risk of contamination, which affects the examination results. The current examination result is an error." is output. In a case where the degree of the risk is the level 2, the final determination as to whether or not to adopt the examination result is left to the user, and the caution is given for the examination result.

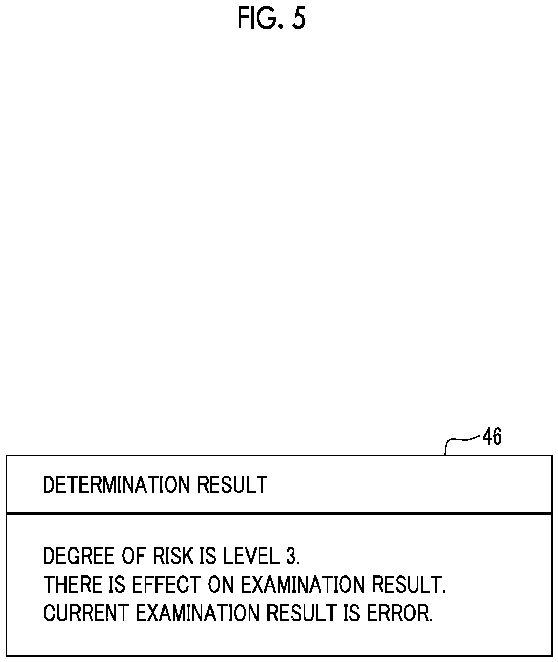

The display screen 46 shown in Fig. 5 is an example of a display screen which displays the determination result in a case where the degree of the risk is the level 3. As shown in Fig. 5, the processor 16 issues a warning indicating that the examination result of the specimen 22 is an error.

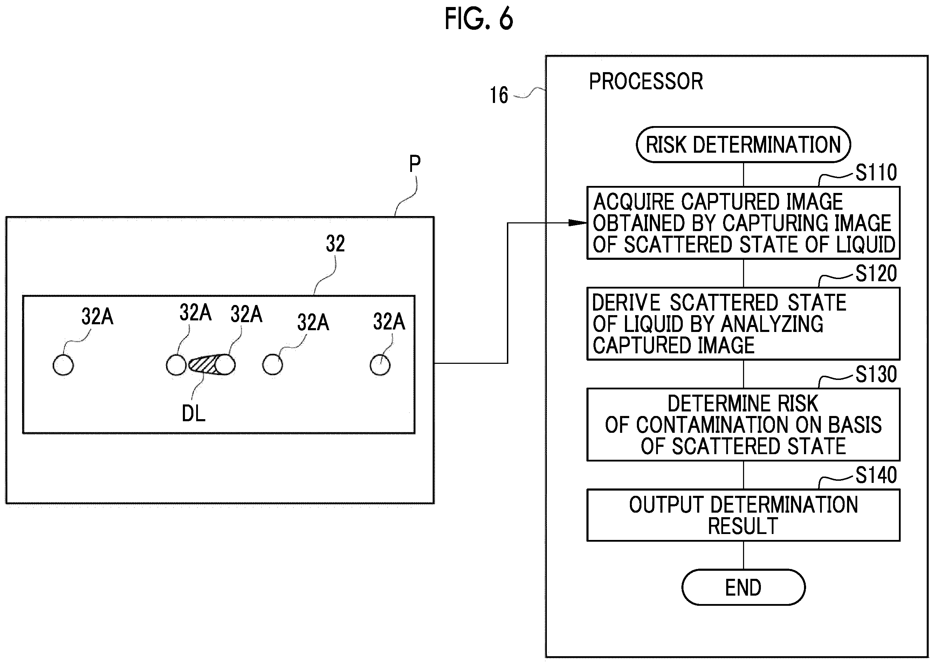

Hereinafter, operations of the above-mentioned configuration will be described with reference to Fig. 6. In a case where the examination is performed, the examination apparatus 10 is loaded with the specimen collection container 20 accommodating the specimen 22 to be examined and the cartridge 30. In addition, a plurality of sets of the specimen collection container 20 and the cartridge 30 may be collectively loaded into the examination apparatus 10 such that the examination apparatus 10 can continuously process examinations for the plurality of specimens 22.

The processor 16 operates the dispensing mechanism 12 in a preset examination procedure to mix the specimen 22 and the reagent S by using the cartridge 30. Further, the processor 16 executes the risk determination process in parallel with the progression of the examination procedure.

In the risk determination process, first, after the nozzle 12A of the dispensing mechanism 12 perforates the sealing film 32 of the cartridge 30, the processor 16 causes the camera 14 to capture an image of the scattered state of the liquid DL on the front surface of the sealing film 32 in a case where the nozzle 12A perforates the sealing film 32. The camera 14 outputs the captured image P to the processor 16. Thereby, the processor 16 acquires the captured image P obtained by capturing the image of the scattered state of the liquid DL (Step S110).

Next, in Step S120, the processor 16 derives the scattered state of the liquid DL by analyzing the captured image P. In the derivation of the scattered state, as described above, for example, the processor 16 extracts the contour of the scattered liquid DL, obtains the area within the extracted contour, or obtains the interval between the liquid DL and the hole 32A adjacent thereto.

Next, in Step S130, the processor 16 determines the risk of the contamination of the liquid DL, on the basis of the derived scattered state of the liquid DL. In the risk determination, the processor 16 determines which degree of risk in the level 1 to 3 corresponds to the derived scattered state of the liquid DL, by referring to the determination condition table 41.

Next, in step 140, the processor 16 outputs the determination result of the risk. The touch panel display 18 displays the determination result. The determination result includes an effect of the determined degree of the risk on the examination result, in addition to the degree of risk in the levels 1 to 3. As described above, in a case where the degree of the risk is the level 1, a message such as "No effect on the current examination result" is displayed. In a case where the degree of the risk is the level 3, a message such as "The examination result is affected and the current examination result is an error" is displayed as shown in Fig. 5.

As described above, the examination apparatus 10, which is an example of the embodiment, includes the dispensing mechanism 12 (an example of a perforating mechanism) that perforates the sealing film 32 of the cartridge 30, which is a liquid container, by the nozzle 12A (an example of a perforating tool), and the camera 14 that captures an image of the scattered state of the liquid DL on the front surface of the sealing film 32 in a case where the nozzle 12A perforates the sealing film 32 and outputs a captured image P (an example of an image which has been captured). Thereby, a user is able to ascertain the adverse effect caused by the scattering of the liquid DL, such as the risk of contamination. In the above-mentioned example, the risk of contamination has been described as the unfavorable effect of the scattering of the liquid DL, but the unfavorable effect of the scattering of the liquid DL may be other than the risk of contamination, and for example, the contamination situation due to the scattering of the liquid DL in the examination apparatus 10 may be included.

In the above-mentioned example, the example has been described in which the processor 16 derives the scattered state of the liquid DL by analyzing the captured image P, but the captured image P may be simply output without analyzing the captured image P. In a case where the captured image P is simply output, the user is able to ascertain the scattered state of the liquid DL. In such a case, the user visually checks the captured image P in the risk determination.

In the present example, the processor 16 determines a method of deriving the scattered state of the liquid DL from the captured image P and a method of determining the risk of the contamination of the liquid DL on the basis of the scattered state, in a rule-based manner using conditions such as the area and the interval. However, instead of determining the determination target in the rule-based manner, the determination target may be determined by using a machine learning model. In such a case, for example, the captured image P is used as input data to output the degree of the risk of contamination to the machine learning model. The machine learning model is a pre-trained model that is trained in advance using supervised training data, which is a combination of the captured image P and correct answer data of the degree of the risk.

In the above-mentioned example, the processor 16 derives the scattered state of the liquid DL by analyzing the captured image P. Thereby, mechanical determination can be made, and a stable determination result can be obtained as compared with the visual determination.

Further, in the above-mentioned example, the processor 16 determines the risk of the contamination of the liquid DL on the basis of the scattered state derived by the image analysis. Therefore, it is possible to determine the risk more accurately than the determination through the visual examination.

Further, in the above-mentioned example, the processor 16 outputs the determination result of the risk to the touch panel display 18. Thereby, it is possible to check the determination result of the risk determined by the examination apparatus 10.

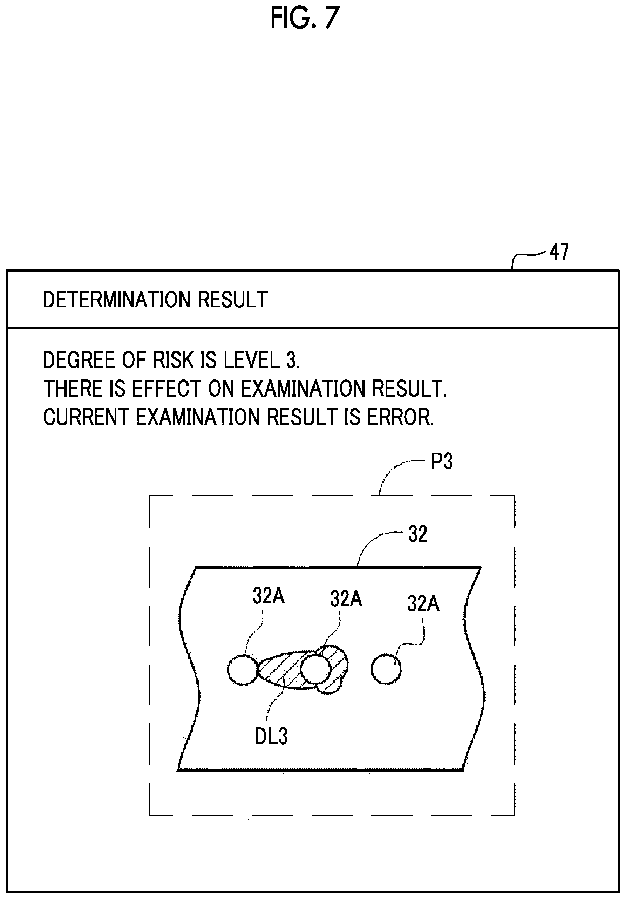

Further, as a modification example, as shown in Fig. 7, the processor 16 may output a display screen 47, on which the captured image P (an example of the captured image P3 in Fig. 7) serving as a basis for the determination is displayed, to the touch panel display 18 in addition to the determination result. In a case where the captured image P is displayed, the user is able to specifically ascertain what kind of scattered state is present.

Further, regarding the timing of the risk determination of contamination, for example, the processor 16 acquires the captured image P each time the perforation is performed by the nozzle 12A, and determines the risk each time the captured image P is acquired. In such a case, the determination result may be output each time the risk is determined, or the determination results for a plurality of times based on the plurality of captured images P may be collectively output. In a case of collectively outputting the plurality of determination results, it is preferable to output the captured image P, which is the basis of such determination as shown in Fig. 7, for each determination result.

Further, the processor 16 may acquire the captured image P in a stage where all the perforations into the cartridge 30 are ended to determine the risk of contamination, and may perform the determination only once on the basis of the acquired captured image P.

As a matter of course, since the scattered state of the liquid DL may change over time, the scattered state of the liquid DL can be more accurately derived by determining the risk each time one hole 32A is perforated. On the other hand, in a case where a temporal change in the scattered state of the liquid DL is small, there is also an advantage in reducing the load of the process of the processor 16 by performing the risk determination only once at a stage where all the perforations are completed. In such a manner, various merits are considered depending on how to set a timing of the risk determination. Therefore, the timing of the risk determination may be changed by the setting. In such a manner, it is easy to appropriately reflect the user's request.

Further, in the above-mentioned example, as shown in Fig. 5, the processor 16 issues a warning that the examination result of the specimen 22 is an error in a case where the degree of the risk is greater than the preset degree. Thereby, it is possible to issue an appropriate warning to the user in a case where the risk of contamination has a large effect on the examination result.

In the above-mentioned example, the effect on the examination result is output in not only the case of the risk degree of the level 3 but also the cases of the risk degrees of the level 1 and the level 2, but a warning that the examination result is an error may be output only in a case where the risk degree is greater than the preset degree as in the case of the level 3.

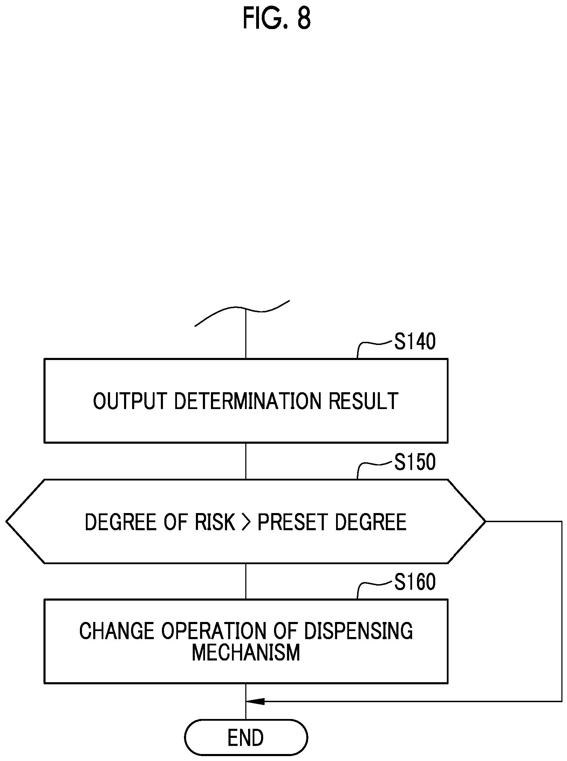

Further, instead of or in addition to the above-mentioned example, as shown in Fig. 8, the processor 16 may perform a control of changing an operation of the dispensing mechanism 12 (an example of the perforating mechanism) in a case where the degree of the risk is greater than the preset degree. Fig. 8 is a modification example of the flowchart shown in Fig. 6, and shows a change part after Step S140. In Fig. 8, after Step S140 of outputting the determination result, the processor 16 proceeds to Step S150 and determines whether or not the degree of the risk is greater than the preset degree (for example, level 2). In a case where it is determined that the degree of the risk is greater than the preset degree, the operation of the dispensing mechanism 12 from next time is changed.

A high degree of the risk means that the liquid DL is relatively scattered as in the captured image P3 shown in Fig. 4. The cause of the scattering of the liquid DL is considered to be also relating to the operation of the nozzle 12A. Therefore, for example, the scattering of the liquid DL is reduced through change in the operation of the dispensing mechanism 12 as a perforating mechanism, such as a decrease in the operation speed of the nozzle 12A as a perforating tool.

Further, in the present example, as the perforating tool, the nozzle 12A that performs suction and discharge of the liquid is used. In such a case, the processor 16 may perform a control of changing the suction amount or the discharge amount of liquid suctioned or discharged through the nozzle 12A as a control of changing the operation of the dispensing mechanism 12 that is an example of the perforating mechanism. For example, in a case where the reagent S is scattered as the liquid DL, in a case where it is considered that the appropriate amount of the reagent S to be dispensed is insufficient due to the scattering, the control of increasing the discharge amount of the reagent S is performed. Further, it is considered that the suction amount is controlled to be increased by re-suctioning the reagent S.

Further, in the above-mentioned example, the cartridge 30 (an example of the liquid container) has the plurality of cells R (an example of the plurality of accommodating portions) for accommodating the plurality of types of liquids (the specimen 22 and the reagent S in the present example). In a case where the plurality of cells R are provided as in the cartridge 30, the risk of contamination due to scattering of the liquid DL is higher than that in a case where one cell R is provided. The technology of the present disclosure is particularly effective in a case of using such a cartridge 30.

Further, in the above-mentioned example, the cartridge 30 includes the specimen 22 and the reagent S as the plurality of types of liquids to be accommodated in the plurality of cells R. In a case where the specimen 22 and the reagent S are included, the risk of contamination is higher than that in a case where only one of the specimen 22 and the reagent S is included. The technology of the present disclosure is particularly effective in a case of using such a cartridge 30.

Further, in the above-mentioned example, the cartridge 30 includes the plurality of types of the reagents S as the plurality of types of liquids. In a case where the plurality of types of the reagents S are used, the risk of contamination is higher than that in a case where one type of the reagent S is used. The technology of the present disclosure is particularly effective in a case of using such a cartridge 30.

In the above-mentioned example, the immunological analysis apparatus that detects the examination target substance included in the specimen by using the antigen-antibody reaction was described as an example of the examination apparatus 10. As a matter of course, the technology of the present disclosure can be used for an examination apparatus other than the immunological analysis apparatus. Further, the chemiluminescent immunoassay method has been described as an example of the method of detecting the examination target substance, but the present disclosure is not limited to this method, and may be applied to other methods.

Further, in the above-mentioned embodiment, as a hardware structure of the processor, various processors shown below can be used. Various processors include a programmable logic device (PLD) that is capable of changing a circuit configuration after manufacturing, such as a field-programmable gate array (FPGA), and a dedicated electric circuit that is a processor having a circuit configuration dedicatedly designed for executing specific process, such as an application specific integrated circuit (ASIC), in addition to a CPU that is a general-purpose processor configured to execute software (program) to function as various processing units.

Further, the above-mentioned process may be executed by one of various processors or may be executed by a combination of two or more processors (for example, a combination of a plurality of FPGAs or a CPU and an FPGA) of the same type or different types. The plurality of processing units may be configured of one processor. As an example where a plurality of processing units are composed of one processor, there is a form in which a processor that realizes all functions of a system including a plurality of processing units into one integrated circuit (IC) chip is used, such as system-on-chip (SOC).

Further, the hardware structure of the processors is, more specifically, an electric circuit (circuitry) in which circuit elements such as semiconductor elements are combined.

Furthermore, the technology of the present disclosure is applied to not only the operation program of the examination apparatus but also a non-transitory computer readable storage medium (a USB memory, a digital versatile disc (DVD)-read only memory (ROM), or the like) storing the operation program of the examination apparatus.

It should be noted that the description contents and shown contents having been described above are the detailed description of parts according to the technology of the present disclosure, and are merely an example of the technology of the present disclosure. For example, description relating to the above-mentioned configurations, functions, operations, and effects is description relating to an example of configurations, functions, operations, and effects of the parts according to the embodiment of the technology of the present disclosure. Therefore, unnecessary parts may be deleted or new elements may be added or replaced in the above descriptions and illustrations without departing from the gist of the technology of the present disclosure. Further, the description of common technical knowledge and the like, which allow the technology of the present disclosure to be embodied and do not need to be particularly described, is omitted in the description contents and shown contents, which have been described above, to avoid complication and to facilitate the understanding of parts according to the technology of the present disclosure.

All documents, patent applications, and technical standards described in the present specification are incorporated into the present specification by reference to the same extent as in a case where the individual documents, patent applications, and technical standards were specifically and individually stated to be incorporated by reference.

The disclosure of JP2022-046016 filed on March 22, 2022 is incorporated herein by reference in its entirety.

## Claims

1. An examination apparatus that examines a specimen using a liquid container which accommodates a liquid and of which an opening portion is sealed with a sealing film, the examination apparatus comprising:
a perforating mechanism that perforates the sealing film with a perforating tool; and
a camera that captures an image of a scattered state of the liquid on a front surface of the sealing film in a case where the sealing film is perforated with the perforating tool, and outputs a captured image.

2. The examination apparatus according to claim 1, further comprising a processor that is configured to derive the scattered state of the liquid by analyzing the image.

3. The examination apparatus according to claim 2,
wherein the processor is configured to determine a risk of contamination of the liquid on the basis of the scattered state.

4. The examination apparatus according to claim 3,
wherein the processor is configured to output a determination result of the risk.

5. The examination apparatus according to claim 3 or 4,
wherein the processor is configured to issue a warning that an examination result of the specimen is an error in a case where a degree of the risk is greater than a preset degree.

6. The examination apparatus according to any one of claims 3 to 5,
wherein the processor is configured to perform a control of changing an operation of the perforating mechanism in a case where a degree of the risk is greater than a preset degree.

7. The examination apparatus according to claim 6,
wherein the processor is configured to perform, as the control of changing the operation of the perforating mechanism, a control of changing a suction amount or a discharge amount of the liquid in a case where the perforating tool is a nozzle that performs suction and discharge of the liquid.

8. The examination apparatus according to any one of claims 1 to 7,
wherein the liquid container has a plurality of accommodating portions that accommodate a plurality of types of liquids as the liquid.

9. The examination apparatus according to claim 8,
wherein the liquid includes at least one of the specimen or a reagent which is used for examination of the specimen.

10. The examination apparatus according to claim 9,
wherein the reagent includes a plurality of types of reagents.

11. The examination apparatus according to any one of claims 1 to 10,
wherein an examination target substance included in the specimen is detected by using an antigen-antibody reaction.
